# EUROPÄISCHE PATENTSCHRIFT

(11) **EP 3 897 444 B1**
(45) Veröffentlichungstag und Bekanntmachung des Hinweises auf die Patenterteilung: **15.10.2025**
(21) Anmeldenummer: 19836479.6
(22) Anmeldetag: 17.12.2019
(51) Int. Cl.: A61B 90/70, A61L 2/26

(54) **ENERGIEGEWINNUNGSVORRICHTUNG**
ENERGY RECOVERY DEVICE
DISPOSITIF DE PRODUCTION D'ÉNERGIE

(30) Priorität: 19.12.2018 DE 102018132962
(43) Veröffentlichungstag der Anmeldung: 27.10.2021
(73) Patentinhaber: Aesculap AG, 78532 Tuttlingen (DE)
(72) Erfinder: LENZENHUBER, Frederick, 78532 Tuttlingen (DE)
(74) Vertreter: Winter, Brandl - Partnerschaft mbB
(86) Internationale Anmeldenummer: PCT/EP2019/085746
(87) Internationale Veröffentlichungsnummer: WO 2020/127340

(56) Entgegenhaltungen:
- DE-A1- 3 320 076
- GB-A- 2 516 806
- KR-A- 20130 031 171
- US-A1- 2010 121 141
- US-A1- 2014 265 336

## Beschreibung

Die Erfindung betrifft eine Energiegewinnungs-/Energieumwandlungsvorrichtung für einen Energiespeicher, und bezieht sich insbesondere auf eine Energiegewinnungs-/Energieumwandlungsvorrichtung für einen Energiespeicher mit einem Modul zum kabellosen und/oder kabelgebundenen Laden des Energiespeichers unter Nutzung eines in einem Gerät vorhandenen Fluidstroms.

### Stand der Technik

Zunehmend gelangen kabellose Systeme, beispielsweise akkumulatorbetriebene Systeme mit ankoppelbaren Energiespeichern, wie etwa Motorensysteme, chirurgische Instrumente im Bereich der Medizin und dergleichen, auf den Markt, so dass ein allgemeiner Trend weg von kabelgebundenen Systemen und hin zu kabellosen bzw. so genannten Stand-Alone Systemen mit einem Energiespeicher ("Akku") zu verzeichnen ist. Diese Energiespeicher müssen mit elektrischer Energie versorgt werden, um die Verfügbarkeit der von ihnen mit Energie belieferten Produkte zu gewährleisten.

Nachteilig ist hierbei, dass energiespeicherbasierte Produkte und Systeme, wie beispielsweise motorenbestückte Handstücke im Bereich der Medizin, vor einem jeweils beabsichtigten Einsatz zunächst geladen werden müssen.

Eine konventionelle Lösung besteht darin, Handstück und Energiespeicher zu entkoppeln und geladene, wechselbare Energiespeicher an einem Behandlungsort, etwa einem Operationssaal (OP), direkt vorzuhalten. Im OP werden sodann sterilisierte Handstücke mit den Energiespeichern bestückt. Weiter nachteilig sind dabei die zusätzlich erforderliche Handhabung, jeweilige Baugrößen separater Energiespeicher und dergleichen.

In den kommenden Jahren ist eine weitergehende Miniaturisierung entsprechender Systeme und Produkte zu erwarten, wobei kleinere Energiespeicher (Akkus, Powercells und dergleichen) denkbar sind. Derzeit ist bereits absehbar, dass diese Energiespeicher im Handstück integrierbar oder als kleineres System angliederbar sein werden.

In Fällen, in denen solche Systeme und/oder Handstücke vor einer Wiederverwendung aufbereitet werden müssen, wie es in beispielsweise dem medizinischen Bereich häufig der Fall ist, besteht ein Bedarf dahingehend, diese Systeme und/oder Handstücke, d.h. auch deren Energiespeicher oder Akkus, in einem Reinigungs-/Desinfektionsgerät (RDG) aufzubereiten. Eine Zielsetzung besteht mithin in einem Übergang weg von im OP vorgehaltenen Energiespeichern hin zu integrierten Energiespeichern. Ein separat notwendiges Aufladen der Energiespeicher im OP führt dann zwangsläufig zu unnötigen oder unnötig komplizierten Betriebsabläufen und höherem oder gar störendem Aufwand, einhergehend mit entsprechend höheren Kosten.

Druckschriften US 2010/121141 A1, KR 2013 0031171 A sowie DE 33 20 076 A1 offenbaren Fluid-getriebene Turbinen für medizinische Instrumente: So betrifft zunächst US 2010/121141 A1 ein chirurgisches Schneide-Endoskop zum Visualisieren und Behandeln einer Operationsstelle innerhalb eines menschlichen Körpers. Das Schneide-Endoskop verwendet einen langgestreckten röhrenförmigen Körper, der so angepasst ist, dass er durch Körperpassagen eingeführt wird, so dass das distale Ende der Operationsstelle benachbart ist. Dabei wird eine neben dem distalen Ende zur Drehung gelagerte Fluidturbine durch Fluide angetrieben, die durch einen Durchgang gepumpt werden, um die Turbine zu drehen. Die verschiedenen Schneider und Wundausschneidungswerkzeuge können an den distalen Enden befestigt werden, so dass sie von der Turbine gedreht werden.

Ferner offenbart KR 2013 0031171 A ein Wasserversorgungssystem, das mit einer LED-Lichtquelle mit Funktionen zur Desinfektion und Lichttherapie ausgestattet ist, umfasst weiter ein Gehäuse, einen Stromgenerator und einen Luftblasengenerator. Dabei erzeugt der Stromgenerator Strom aus dem Druck und der Strömungsgeschwindigkeit des im Gehäuse fließenden Wassers, um die LED-Lichtquelle zu betreiben.

Ferner beschreibt DE 33 20 076 A1 das Voranbringen von medizinischen Schläuchen und Sonden unter Verwendung von Düsenkörpern. Durch den Einbau nur weniger weiterer technischer Elemente, wie einer Welle und für Fluid gelochten Scheiben, und durch die radiale Einführung von Düsen zur Längsachse können solche Düsenkörper zu Turbinen umgestaltet werden.

### Kurzbeschreibung der Erfindung

Der Erfindung liegt daher als eine Aufgabe zugrunde, eine Energiegewinnungs-/Energieumwandlungsvorrichtung bereitzustellen, die ein Aufladens von Systemen mit Energiespeichern vor der Nutzung im OP ermöglicht und dadurch die Handhabbarkeit und Benutzbarkeit solcher Systeme verbessert.

Darüber hinaus soll die Erfindung das Ernten bzw. "harvesten" und somit Laden von Energiespeichern in einem RDG ermöglichen.

Erfindungsgemäß wird diese Aufgabe durch ein Modul mit den Merkmalen des Anspruchs 1 gelöst. Vorteilhafte Weiterbildungen der Erfindung sind Gegenstand der beigefügten Unteransprüche.

Gemäß einer allgemein der Erfindung zugrunde liegenden Idee sollen Systeme und/oder Produkte durch Gewinnen, d.h. Ernten bzw. "harvesting", von elektrischer

Energie aus einem bestehenden Fluidkreislauf geladen werden, um ein Aufladen der Systeme vor der Nutzung zu ermöglichen. Gleichzeitig wird eine Datenübertragungsschnittstelle bereitgestellt, über welche ein Ladezustand ausgelesen werden kann, oder eine Funktion bereitgestellt, die Ladezustände angebundener Systeme bzw. Produkte nach außen hin meldet.

Ein konkretes Beispiel dieser der Erfindung zugrundeliegenden allgemeinen Idee besteht beispielsweise im medizinischen Bereich darin, dort verwendete (chirurgische) Systeme und/oder Produkte wie etwa Handstücke mit integrierten oder ankoppelbaren Energiespeichern und dergleichen in einem Produktaufbereitungsprozess durch "harvesting", d.h. Gewinnen oder Erzeugen von (elektrischer) Energie in bzw. aus einem bestehenden Fluidkreislauf, beispielsweise einem Wasserkreislauf, eines Reinigungs- und Desinfektionsgeräts (RDG) zu laden, um ein notwendiges Aufladen der Systeme und/oder Produkte vor der Nutzung im OP zu ermöglichen. Eine entsprechend leistende Energiegewinnungs-/Energieumwandlungsvorrichtung kann als ein Modul aufgebaut sein, das mittels einer Datenübertragungsschnittstelle ausgelesen werden kann oder die Ladezustände der angebundenen Produkte nach außen hin meldet.

In anderen Worten beinhaltet - anhand des vorgenannten konkreten Beispiels - die zugrundeliegende allgemeine Idee eine Energiegewinnungsvorrichtung bzw. ein Modul zum kabellosen oder kabelgebundenen Laden von Energiespeichern unter Nutzung des herrschenden Fluiddrucks in einem ohnehin schon für andere Zwecke bestehenden bzw. vorhandenen Fluidstrom oder Fluidkreislauf (beispielsweise dem Wasserkreislauf in einem Reinigungs- und Desinfektionsgerät). Es versteht sich unmittelbar, dass weder die allgemeine Idee noch die Erfindung auf den medizinischen Bereich und dort insbesondere einen Fluidkreislauf eines RDG und entsprechende Systeme und/oder Produkte beschränkt sind. Entscheidend bei der vorliegenden Erfindung ist, dass elektrische Energie quasi als Abfallprodukt aus bereits vorhandenen Energiesystemen wie Fluidströmungen zum Kühlen/Betreiben/Reinigen etc. zurückgewonnen wird, welche dann nutzbringend zum Aufladen von Energiespeichern chirurgischer Instrumente verwendet werden kann.

Vorteile, die aus der Möglichkeit, Systeme und/oder Produkte durch Gewinnen von Energie aus einem Fluidkreislauf, beispielsweise während eines Aufbereitungsprozesses in einem RDG, zu laden, erfindungsgemäß unmittelbar resultieren, beinhalten eine signifikante Einsparung von Vorbereitungszeit, einen reduzierten Handhabungsaufwand am Einsatz- bzw. Verwendungsort wie etwa einem OP, die sofortige Einsetzbarkeit von Systemen und Produkten am Einsatz- bzw. Verwendungsort, und die Möglichkeit, Ladezustände über eine Datenübertragungsschnittstelle einzusehen, beispielsweise über eine Anwendung für mobile Geräte, eine Software auf einem PC und dergleichen.

Im Einzelnen wird die Aufgabe gelöst durch eine Energiegewinnungs-/Energieumwandlungsvorrichtung für einen aufladbaren Energiespeicher, wobei die Energiegewinnungsvorrichtung dafür angepasst ist, an/in einer von einem Fluid durchströmten Fluidleitung eines bestehenden Fluidkreislaufs in einem Gerät angeordnet zu werden und der Fluidstrom in der Fluidleitung primär einem vorbestimmten Zweck dient, der sich von dem der Energiegewinnung unterscheidet. Die Energiegewinnungsvorrichtung ist dazu angeordnet, aus der Bewegungsenergie des Fluidstroms elektrische Energie zur Aufladung des aufladbaren Energiespeichers zu gewinnen.

Bevorzugt ist die Energiegewinnungsvorrichtung modulförmig aufgebaut und dazu angeordnet, mittels eines Energie-Harvesting-Prozesses auf der Grundlage des Fluiddrucks in dem bestehenden Fluidkreislauf des Geräts elektrische Energie zu erzeugen und mit der so erzeugten Energie den Energiespeicher zu laden.

Weiter bevorzugt beinhaltet die Energiegewinnungsvorrichtung eine Umwandlungseinheit zur Umwandlung des Fluiddrucks des Fluidstroms bzw. der Strömungsenergie des Fluids in eine Rotationsbewegung beispielsweise einer Turbine und eine Generatoreinheit zur Umwandlung der Rotationsbewegung in erzeugte elektrische Energie.

Bevorzugt beinhaltet die Umwandlungseinheit zur Umwandlung des Fluiddrucks/der Strömungsenergie in eine Rotationsbewegung ein Rotorelement mit einer vorbestimmten Geometrie, das in einem Gehäuse der Energiegewinnungsvorrichtung und in dem Fluidstrom liegend drehbar gelagert ist.

Vorteilhaft ist dabei, wenn die vorbestimmte Geometrie schaufelradförmig oder schneckenförmig ist. Alternativ ist aber auch eine sogenannte Kugelturbine denkbar bestehend aus einer Kugel, längs deren Äquatorlinie umfangsbeabstandete Taschen ausgenommen sind, die als Drucksammelmulden zum rotorischen Antrieb der Kugel dienen. Die Kugel selbst kann von einem Drehschaft zentral durchdrungen sein, der beidseits der Kugel drehgelagert ist.

Außerdem bevorzugt kann in der Energiegewinnungsvorrichtung die Umwandlungseinheit zur Umwandlung des Fluiddrucks in eine Rotationsbewegung ein während eines Durchspülvorgangs von der Fluidströmung angetriebener, als Generator arbeitender Elektromotor sein, der einen eine vorbestimmte Geometrie aufweisenden Rotor beinhaltet, und dazu konfiguriert ist, in Zusammenwirkung mit einem Statorelement elektrischen Strom zu erzeugen, mittels dem der Energiespeicher vermittels einer Induktionseinrichtung zum induktiven Laden des Energiespeichers oder vermittels eines Ladekabels zum kabelgebundenen Laden des Energiespeichers aufladbar ist.

Bevorzugt beinhaltet die Energiegewinnungsvorrichtung eine elektronische Steuerung, die in einem Gehäuse der Energiegewinnungsvorrichtung auf einer Leiterplatte angeordnet ist.

Vorteilhaft ist die Energiegewinnungsvorrichtung dazu konfiguriert, einen Füllstand des Energiespeichers zu erfassen und bei Erfassen eines vorbestimmten (maximalen) Füllstands einen Bypass zu öffnen, wobei der Bypass dazu angeordnet ist, im geöffneten Zustand den Fluidstrom an der Umwandlungseinheit vorbeizuführen, und wobei in diesem Fall die Umwandlungseinheit dazu konfiguriert ist, sowohl ihre Rotation als auch die Erzeugung von Energie zur Aufladung des Energiespeichers zu beenden.

Bevorzugt ist die von der Generatoreinheit erzeugte elektrische Energie mittels Induktion über eine eine Induktionsleitung umfassende flexible Leiterplatte an den Energiespeicher und/oder ein Produkt übertragbar.

Alternativ bevorzugt ist die von der Generatoreinheit erzeugte Energie kabelgebunden über ein Ladekabel an den Energiespeicher übertragbar.

Auch bevorzugt weist in die Energiegewinnungsvorrichtung das Modul, eine Vorrichtung zur Ermittlung eines Ladezustands eines angebundenen Energiespeichers und eine Datenübertragungsschnittstelle auf, über welche das Modul auslesbar ist oder den Ladezustand nach außen hin kommuniziert.

Ein die Energiegewinnungsvorrichtung beinhaltendes Modul ist darstellbar, wobei das Modul zur Verwendung in einem Aufbereitungsprozess für Produkte, Systeme und/oder Instrumente mit einem integrierten oder ankoppelbaren, wechselbaren und aufladbaren Energiespeicher und dazu konfiguriert ist, während des Aufbereitungsprozesses in einer Aufbereitungsvorrichtung gehalten oder gelagert zu werden. Das Modul ist gekennzeichnet durch eine Energiegewinnungsvorrichtung wie vorstehend beschrieben; einen Anschluss zur Verbindung des Moduls mit einem Fluidkreislauf der Aufbereitungsvorrichtung; eine Leitungsverbindung zur Führung eines an dem Anschluss in das Modul einströmenden Fluids durch das Modul; einen Portabschnitt; zumindest einen Anschlussport für zumindest ein Produkt, System und/oder Instrument, der an dem Portabschnitt angeordnet ist und dazu konfiguriert ist, ein Produkt, System oder Instrument derart zu halten, dass dieses von über den Anschlussport einströmendem Wasser durchströmt werden kann; und eine Induktionsvorrichtung, die flächig unter dem Anschlussport in einem Bereich angeordnet ist, in dem der Energiespeicher (nicht dargestellt) des an dem Anschlussport angeschlossenen Produkts, Systems und/oder Instruments im angeschlossenen Zustand zu liegen kommt, und dazu konfiguriert ist, den Energiespeicher vermittels von der Energiegewinnungsvorrichtung aus dem Fluidstrom geernteter Energie während des Betriebs der Aufbereitungsvorrichtung zu laden. Ferner kann eine elektronische Steuerung zur Steuerung von Betriebsabläufen an dem Modul vorgesehen sein.

In einem praxisnahen Fall ist bevorzugt das Gerät ein Reinigungs-/Desinfektionsgerät (RDG) zur Aufbereitung medizinischer Instrumente, Systeme und/oder Produkte, wobei die medizinischen Instrumente, Systeme und/oder Produkte zumindest ein Handstück mit einem an dieses ankoppelbaren und/oder in dieses integrierten, aufladbaren Energiespeicher umfassen, wobei der Energiespeicher als Akkumulator oder als Powercell konfiguriert ist und dazu ausgelegt ist, zusammen mit dem Handstück in dem Reinigungs-/Desinfektionsgerät aufbereitet zu werden.

Dann weiter bevorzugt wird ein Reinigungs-/Desinfektionsgerät (RDG) zur Aufbereitung medizinischer Instrumente, Systeme und/oder Produkte, wobei die medizinischen Instrumente, Systeme und/oder zumindest ein Handstück mit einem an dieses ankoppelbaren und/oder in dieses integrierten, aufladbaren Energiespeicher umfassen, wobei der Energiespeicher als Akkumulator oder Powercell konfiguriert ist und dazu ausgelegt ist, zusammen mit dem Handstück in dem Reinigungs-/Desinfektionsgerät aufbereitet zu werden. In dem Reinigungs-/Desinfektionsgerät ist eine Energiegewinnungsvorrichtung nach einem der vorangehenden Ansprüche dazu konfiguriert ist, a) an einer vorbestimmten Lagerung oder einem vorbestimmten Siebkorb des Reinigungs-/Desinfektionsgeräts gehalten zu werden, oder b) als ein an einem Spülrack des Reinigungs-/Desinfektionsgeräts integriertes Modul mit einer Induktionsvorrichtung zur drahtlosen Übertragung von Energie mittels Induktion oder einer Ladekabelanordnung zur kabelgebundenen Übertragung von Energie zum Laden eines an dem Reinigungs-/Desinfektionsgerät angeschlossenen Produkts ausgebildet zu sein, oder c) als ein an einer vorbestimmten Lagerung des Reinigungs-/Desinfektionsgeräts integriertes Modul mit einer Induktionsvorrichtung zur drahtlosen Übertragung von Energie mittels Induktion oder einer Ladekabelanordnung zur kabelgebundenen Übertragung von Energie zum Laden eines in dem Reinigungs-/Desinfektionsgerät aufgenommenen und mit zumindest einem Energiespeicher ausgestatteten Instrumentariums oder eines an einem Schlauchabschnitt des Reinigungs-/Desinfektionsgerät angeschlossenen Produkts ausgebildet zu sein.

In diesem Fall kann das Modul auch dauerhaft in dem Reinigungs-/Desinfektionsgerät (RDG) verbaut und mit zumindest einem spritzwasserdurchlässige Ladepad und/oder zumindest einem Ladekabel verbunden sein, wobei das Ladepad dazu konfiguriert ist, zumindest ein Produkt mit zumindest einem Energiespeicher, das über dem Ladepad gelagert ist, induktiv mit der von dem Modul erzeugten Energie zu laden, und das Ladekabel dazu konfiguriert ist, zumindest ein einzelnes, über das Ladekabel angeschlossene Produkt mit einem Energiespeicher über das Ladekabel mit der von dem Modul erzeugten Energie zu laden.

### Kurzbeschreibung der Figuren

Die Erfindung wird nachstehend unter Bezugnahme auf die beigefügte Zeichnung näher beschrieben. Es zeigen:
Fig. 1 eine schematische Darstellung eines Prinzips der Energiegewinnung unter Nutzung der Energie bzw. des Drucks eines Fluidstroms in einem Schlauchabschnitt eines Geräts gemäß einem Ausführungsbeispiel;
Fig. 2 eine schematische Darstellung eines an einem Spülrack integrierten Moduls in Wirkverbindung mit einer Induktionseinrichtung zum Laden eines jeweiligen Energiespeichers einer Vielzahl von Produkten bei einer Energiegewinnungsvorrichtung gemäß dem Ausführungsbeispiel;
Fig. 3 eine schematische Darstellung eines in einem Siebkorb angebrachten Moduls in Wirkverbindung mit einer Induktionseinrichtung zum Laden eines Energiespeichers eines einzelnen Produkts bei einer Energiegewinnungsvorrichtung gemäß dem Ausführungsbeispiel;
Fig. 4 in einer schematischen Darstellung in Verbindung mit Fig. 3 beschriebene Zusammenhänge;
Fig. 5 eine schematische Darstellung eines in einem Reinigungs-/Desinfektions-Geräts dauerhaft verbauten Moduls mit Ladepads bei einer Energiegewinnungsvorrichtung gemäß einem Ausführungsbeispiel;
Fig. 6 eine schematische Darstellung einer Nutzung eines in einem Produkt verbauten Elektromotors als Generator zum Laden eines Energiespeichers des Produkts mit im Generatorbetrieb erzeugter Energie bei einer Energiegewinnungsvorrichtung gemäß einem anderen Ausführungsbeispiel; und
Fig. 7 eine schematische Darstellung der Nutzung des in einem Produkt verbauten Elektromotors als Generator zum Laden des Energiespeichers des Produkts mit im Generatorbetrieb erzeugter Energie bei einer Energiegewinnungsvorrichtung gemäß dem anderen Ausführungsbeispiel.

In den Figuren bezeichnen gleiche Bezugszeichen gleiche oder zumindest äquivalente Teile und Komponenten. Zweckmäßig wird insoweit eine mehrfach redundante Beschreibung solcher Teile und Komponenten weggelassen.

### Detaillierte Beschreibung bevorzugter Ausführungsbeispiele

Bevorzugte Ausführungsbeispiele einer hierin beschriebenen Energiegewinnungsvorrichtung werden nachstehend unter Bezugnahme auf ein an sich bekanntes Reinigungs-/Desinfektions-Gerät (RDG), wie es etwa im medizinischen Bereich zur Aufbereitung wiederverwendbarer Produkte, Systeme und/oder Instrumentarien zum Einsatz kommt, als Beispiel beschrieben. Zur Reinigung und Desinfektion wird bei einem RDG eine Kombination aus mechanischer, thermischer und chemischer Behandlung eingesetzt, die in etwa dem Vorbild einer Spülmaschine entspricht. Gegebenenfalls mit Chemikalien angereichertes, erhitztes Wasser wird mit Druck auf die zu reinigenden Gegenstände aufgebracht. Es versteht sich, dass die Erfindung in keiner Weise auf den medizinischen Bereich, dort verwendete Produkte, Systeme und/oder Instrumentarien, oder die Anordnung in einem oder die Verwendung eines RDG beschränkt ist, sondern entsprechend modifizierte Konfigurationen und Modifikationen für zahlreiche weitere und/oder andere Produkte mit integriertem oder ankoppelbaren (wechselbaren) Energiespeicher und Geräte mit einem Fluidkreislauf denkbar und darstellbar sind.

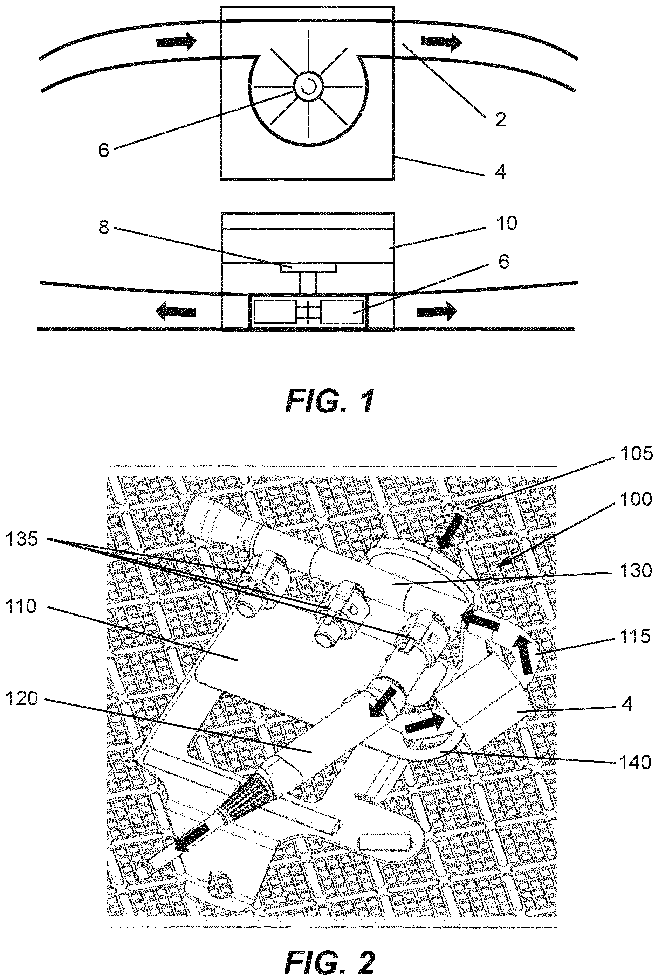

Fig. 1 zeigt in einer Stirnansicht und einer Aufsicht eine schematische Darstellung einer Vorrichtung zur Veranschaulichung eines Prinzips der Energiegewinnung unter Nutzung der Energie bzw. des Drucks eines Fluidstroms in einem Schlauchabschnitt eines Geräts gemäß einem Ausführungsbeispiel. In dem vorliegenden Ausführungsbeispiel ist das Gerät ein an sich bekanntes Reinigungs-/Desinfektions-Gerät (RDG) wie vorstehend beschrieben.

In Fig. 1 bezeichnet ein Bezugszeichen 2 einen Schlauchabschnitt bzw. Leitungsabschnitt eines Fluidkreislaufs in dem Gerät, beispielsweise eines Wasserkreislaufs in dem RDG. Ein Fluidstrom/Wasserstrom mit einem vorbestimmten Fluiddruck/Wasserdruck und/oder einer vorbestimmten Strömungsgeschwindigkeit in dem Schlauchabschnitt 2 ist durch schwarze Pfeile angedeutet.

Ein Bezugszeichen 4 bezeichnet eine Energiegewinnungsvorrichtung gemäß dem Ausführungsbeispiel. Die Energiegewinnungsvorrichtung 4 weist eine Umwandlungseinheit auf, die als eine Welle oder ein Rotor 6 mit einer vorbestimmten Geometrie, beispielsweise einer schaufelradförmigen Geometrie oder einer schneckenförmigen Geometrie, ausgebildet ist und dazu angeordnet und konfiguriert ist, die Längsbewegung des Fluidstroms in dem Schlauchabschnitt 2 in eine Rotationsbewegung umzuwandeln. Der Fluidstrom bzw. dessen Druck und/oder Geschwindigkeit beaufschlagt den Rotor 6 und versetzt diesen in Rotation. Zur Umwandlung der Rotationsbewegung des Rotors 6 in Energie, d.h. zur Erzeugung von Energie, ist eine Generatorvorrichtung 8 angeordnet und konfiguriert. Die Generatorvorrichtung 8 ist mit einer elektronischen Steuerung 10 gekoppelt, die auf einer Leiterplatte (nicht dargestellt) angeordnet sein kann und unter anderem dazu konfiguriert ist, zumindest die Energiegewinnung und die Verteilung der gewonnenen Energie sowie deren vorbestimmte Umsetzung Ausleitung aus der Energiegewinnungsvorrichtung 4 zu steuern. Das Gesamtsystem wie vorstehend beschrieben ist fluiddicht in einem Gehäuse aufgenommen.

Praktisch erfolgt somit bei der Energiegewinnung unter Nutzung der Energie bzw. des Drucks eines Fluidstroms ein sogenanntes Ernten oder "Harvesten" von Energie, und wird dabei der Fluidstrom bzw. der Wasserdruck desselben genutzt, um eine Einheit (die Umwandlungseinheit bzw. deren Rotor 6) anzutreiben, welche den Wasserdruck in dem Schlauchabschnitt 2 in eine Rotationsbewegung umwandelt, beispielsweise mittels eines Schaufelrads, einer Schneckenwelle oder dergleichen. Zur Umwandlung der Rotationsbewegung in Energie wird ein Generator (die Generatorvorrichtung 8) verwendet. Eine elektronische Steuerung 10 befindet sich z.B. auf der Leiterplatte. Das Gesamtsystem ist gehäust.

Fig. 2 zeigt eine schematische Darstellung eines an einem Spülrack des RDG integrierten Moduls 100 in Wirkverbindung mit einer Induktionsvorrichtung 110 zum Laden eines Energiespeichers eines Produkts, Systems oder Instruments 120 bei einer Energiegewinnungsvorrichtung gemäß dem Ausführungsbeispiel. Unter einem Spülrack ist hierin eine Art Ablage vergleichbar mit einem an sich bekannten Spülkorb in dem RDG zu verstehen, auf oder in welchen aufzubereitende Teile auflegbar oder einlegbar sind. Das Spülrack ist in Fig. 2 als ausschnittsweise Hintergrundstruktur angedeutet.

Das Modul 100 ist auf das Spülrack auflegbar konfiguriert, wobei Halterungen oder Fixierungsmöglichkeiten bereitgestellt sein können. Das Modul 100 weist einen Anschluss 105 auf zur Verbindung desselben mit dem Wasserkreislauf des RDG. Pfeile in Fig. 2 deuten einen Strömungsweg von im Betrieb des RDG durch das Modul 100 strömendem Wasser an. Wie in Fig. 2 gezeigt ist, strömt Wasser an dem rückwärtigen Anschluss 105 in das Modul 100 ein und wird über eine Leitungsführung 115 (die in diesem Ausführungsbeispiel den Schlauchabschnitt 2 beinhaltet, aber auch zumindest streckenweise oder zur Gänze starr ausgeführt sein kann) zu einem Portabschnitt 130 geführt. Der Portabschnitt 130 weist eine Vielzahl von Anschlussports 135 für Produkte, Systeme und/oder Instrumente auf. In diesem Ausführungsbeispiel sind beispielsweise 3 Anschlussports 135 bereitgestellt, und ist gemäß Fig. 2 an einem der Anschlussports 135 ein Handstück 120 als eines eines Produkts, Systems oder Instruments derart gehalten, dass es von über den entsprechenden Anschlussport 135 einströmendem Wasser durchströmt werden kann.

In der Leitungsführung 115 ist ferner die in Fig. 1 dargestellte Energiegewinnungsvorrichtung bzw. Energiegewinnungseinheit 4 so angeordnet, dass sie von durch die Leitungsführung 115 fließendem Wasser durchströmt wird und die vorstehend unter Fig. 1 beschriebene Wirkung und Funktion entfaltet. Das heißt, dass im Betrieb des RDG Wasser durch die Energiegewinnungsvorrichtung 4 strömt, der darin drehbar gelagerte Rotor 6 rotiert und vermittels der Rotation die mit dem Rotor 6 gekoppelte Generatorvorrichtung 8 Energie aus primär der Aufbereitung im RDG (und damit nicht primär der Energiegewinnung) dienenden Wasserströmung erntet bzw. gewinnt.

Die erzeugte Energie wird sodann von der elektronischen Steuerung 10 in beispielsweise eine geeignete Form umgesetzt und über eine Leitungsverbindung bzw. Induktionsleitung 140 an die Induktionsvorrichtung 110 ausgeleitet. Die Induktionsvorrichtung 110 kann als geeignete zusätzliche Peripherie beispielsweise eine flexible Leiterplatte mit einer Induktionsleitung beinhalten. Wie in Fig. 2 gezeigt ist, ist die Induktionsvorrichtung 110 flächig unter den Anschlussports 135 in einem Bereich angeordnet, in dem Energiespeicher (nicht dargestellt) von an den Anschlussports 135 angeschlossenen Produkten, Systemen und/oder Instrumenten im angeschlossenen Zustand zu liegen kommen. Auf diese Weise können diese Energiespeicher vermittels der von der Energiegewinnungsvorrichtung mit aus der Wasserströmung geernteter Energie versorgten Induktionsvorrichtung 135 während des Betriebs des RDG, d.h. während dessen Aufbereitungsbetriebsablaufs bzw. Reinigungs- und Desinfektions-Betriebsablaufs, geladen werden.

Die Energiegewinnungsvorrichtung 4 für einen aufladbaren Energiespeicher ist somit an einer von einem Fluid durchströmten Fluidleitung eines bestehenden Fluidkreislaufs in einem Gerät angeordnet. Der Fluidstrom in der Fluidleitung dient primär einem vorbestimmten Zweck, der sich von dem der Energiegewinnung unterscheidet. Die Energiegewinnungsvorrichtung ist dazu angeordnet, aus dem Fluidstrom Energie zur Aufladung des aufladbaren Energiespeichers zu gewinnen. Das Modul 100 beinhaltet die Energiegewinnungsvorrichtung 4 und eine Induktionsvorrichtung 110, die von in der Energiegewinnungsvorrichtung 4 gewonnener (ge"harvesteter") Energie gespeist wird und dazu konfiguriert ist, sich in ihrem Induktionsfeld befindende Energiespeicher aufzuladen, während das RDG seinen vorbestimmten Betriebsablauf ausführt und währenddessen die Energiegewinnungsvorrichtung 4 Energie aus dem Wasserkreislauf des RDG gewinnt. In anderen Worten wird ge"harvestete" Energie über Induktion und somit geeignete zusätzliche Peripherie, wie z.B. eine flexible Leiterplatte mit einer Induktionsleitung, an die Produkte, Systeme und/oder Instrumente übertragen. Alternativ ist auch eine Ladeanbindung über einen +- Pol-Anschluss und somit über ein Ladekabel denkbar. Das RDG beinhaltet zumindest ein Modul 100, das dazu konfiguriert ist, an zumindest einem Spülrack des RDG gehalten zu werden und mit dem Wasserkreislauf des RDG verbunden zu werden.

In einer Modifikation des vorstehend beschriebenen Ausführungsbeispiels kann vorgesehen sein, dass Produkte, Systeme und/oder Instrumente an die elektronische Steuerung 10 zurückmelden, dass ein vorbestimmter Füllstand bzw. Ladezustand ihres Energiespeichers erreicht ist. In diesen Fall kann die elektronische Steuerung 10 dazu konfiguriert sein, mittels geeigneter Ansteuerung beispielsweise eines Stellglieds oder Ventils mechanisch eine Öffnung einer Bypassvorrichtung zu veranlassen, die dazu angeordnet und konfiguriert ist, den Wasserstrom umzuleiten und an dem Rotor 6 vorbeizuleiten, so dass der Rotor 6 nicht weiter rotiert, infolgedessen die Generatorvorrichtung 8 nicht weiter Energie erzeugt und somit das Laden des Energiespeichers beendet oder zumindest für die Öffnungsdauer der Bypassvorrichtung ausgesetzt wird.

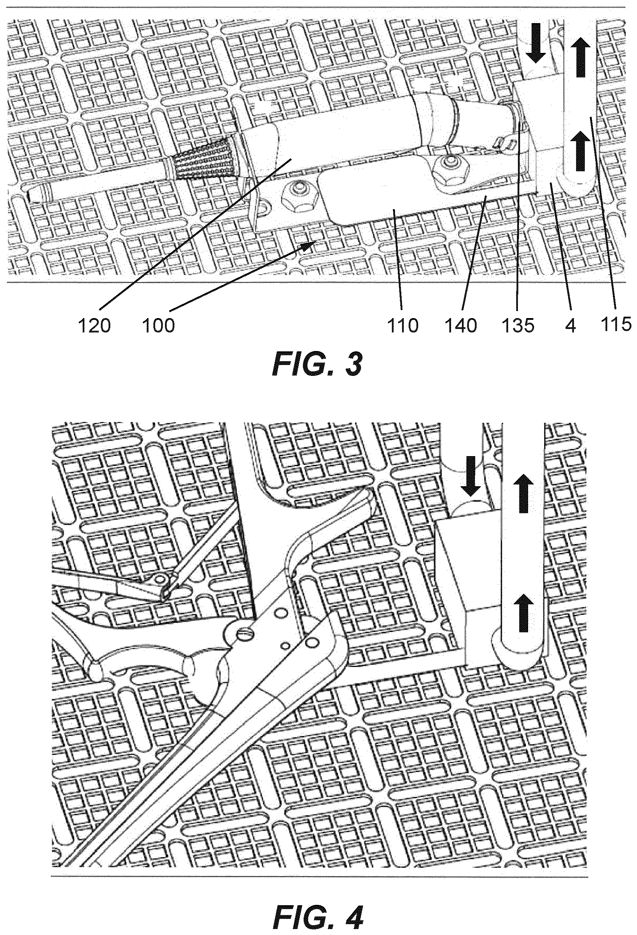

Fig. 3 zeigt eine schematische Darstellung eines in einem Siebkorb angebrachten Moduls 100 in Wirkverbindung mit einer Induktionseinrichtung 135 zum Laden eines Energiespeichers eines Produkts, Systems oder Instruments bei einer Energiegewinnungsvorrichtung 4 gemäß dem Ausführungsbeispiel.

Wie in Fig. 3 gezeigt ist, kann das Modul 100 nach Fig. 2 in einer Modifikation zum Laden eines (einzelnen) Produkts, Systems oder Instruments konfiguriert sein. In diesem Fall kann die Konfiguration des Moduls beispielsweise dahingehend vereinfacht sein, dass der (einzelne) Anschlussport 135 direkt an der Energiegewinnungsvorrichtung 4 verbaut ist. Außerdem kann die Induktionsvorrichtung 110 entsprechend kleiner ausgebildet sein. Es wird angemerkt, dass in Fig. 3 ferner Verschraubungen des Moduls 100 mit dem Spülrack oder Siebkorb zu dessen Fixierung daran oder darin dargestellt sind.

Es versteht sich, dass die in Fig. 3 gezeigte Modifikation nicht auf das Laden eines Produkts, Systems oder Instruments beschränkt ist, und dass beliebige andere Produkte, Systeme und/oder Instrumente mit einem integrierten oder ankoppelbaren, wechselbaren und aufladbaren Energiespeicher in einer Einzelkonfiguration des Moduls 100 aufladbar sind.

Beispielsweise kann mit einer entsprechenden Länge, Größe und/oder Verlegung der Leitungsverbindung 140 bzw. der Induktionsvorrichtung 110, beispielsweise eine Verlegung oder Positionierung an eine von der Energiegewinnungsvorrichtung 4 relativ gesehen weiter entfernte Position, auch der Energiespeicher eines raumgreifenderen, d.h. größeren Produkts, Systems oder Instruments, das zwar Raum beansprucht und daher einen gewissen Abstand zu anderen Komponenten erfordert, als solches aber nicht durchspült zu werden braucht und deshalb keine Ankopplung an einen Anschlussport 135 erfordert, induktiv aufgeladen werden. In einem solchen Fall kann der Anschlussport 135 an der Energiegewinnungsvorrichtung 4 entfallen.

In einer weiteren Modifikation des vorstehenden Ausführungsbeispiels, die größere Produkte, Systeme und/oder Instrumente sowohl in der vorstehenden Einzelkonfiguration (mit wie in Fig. 3 gezeigt einem Anschlussport 135 direkt an der Energiegewinnungsvorrichtung 4) auch bei einem Modul 100 mit mehreren Anschlussports 135 erlaubt, kann zumindest ein Anschluss für die Leitungsverbindung 140 zu der Induktionsvorrichtung 110 steckbar ausgeführt sein und können Anschlussports 135 mechanisch und fluiddicht verschließbar sein, beispielsweise unter Verwendung eines eine Verschlussvorrichtung stellenden Stellglieds und/oder Ventils, das von der elektronischen Steuerung 10 angesteuert wird, oder unter Verwendung einer bei Ankopplung/Abkopplung eines Produkts, Systems oder Instruments selbsttätig öffnenden/schließenden Membran oder Verschlussvorrichtung oder dergleichen.

In diesem Fall kann eine Konfiguration derart sein, dass dann, wenn von der elektronischen Steuerung 10 ein Vorhandensein eines Produkts, Systems oder Instruments und/oder eine mit der Energiegewinnungsvorrichtung 4 verbundene Induktionsvorrichtung 110 erfasst wird, aber eine Nichtbelegung eines entsprechenden Anschlussports 135 erfasst wird, d.h. wenn die elektronische Steuerung erkennt, dass Energiespeicher eines Produkts, Systems oder Instruments zwar aufgeladen werden soll, das Produkt, System oder Instrument aber nicht durchspült werden soll und deshalb nicht mit dem Anschlussport 135 verbunden wurde, die elektronische Steuerung 10 veranlasst, dass der Anschlussport 135 fluiddicht geschlossen wird und Ladeenergie zu der Induktionsvorrichtung 135 geleitet wird, um den entsprechenden Energiespeicher aufzuladen.

In der vorgenannten Konfiguration können raumgreifende Produkte, Systeme und/oder Instrumente, die aufgrund ihrer Größe oder Konstruktionsweise nicht mit dem zumindest einen am Modul 100 oder dem einen an der Energiegewinnungsvorrichtung 4 vorhandenen Anschlussport 135 verbindbar sind oder die nicht durchspülbar und deshalb nicht mit dem am Modul 100 oder an der Energiegewinnungsvorrichtung 4 vorhandenen Anschlussport 135 verbindbar sind, auch dann mit gleichzeitiger Aufladung ihres Energiespeichers aufbereitet werden, wenn das Modul 100 und/oder die Energiegewinnungsvorrichtung 4 über einen oder mehrere Anschlussports 135 verfügt. In anderen Worten bedingt ein raumgreifendes Produkt, System oder Instrument keine spezielle Ausführungsform (jeweils mit/ohne Anschlussport) des Moduls 100 und/oder der Energiegewinnungsvorrichtung 4, sondern können beliebige Produkte, Systeme oder Instrumente unter Verwendung einheitlich aufgebauter Module 100 oder Energiegewinnungsvorrichtungen 4 aufbereitet und gleichzeitig geladen werden. Es wird angemerkt, dass nicht belegte Anschlussports 135 standardmäßig geschlossen sein können, so dass an belegten Ports ein jeweils maximaler Fluid- bzw. Wasserdruck anliegt.

Fig. 4 veranschaulicht in einer schematischen Darstellung die vorstehend in Verbindung mit Fig. 3 beschriebenen Zusammenhänge. Aus Vereinfachungs- und Übersichtlichkeitsgründen sind in Fig. 4 redundante Bezugszeichen weggelassen.

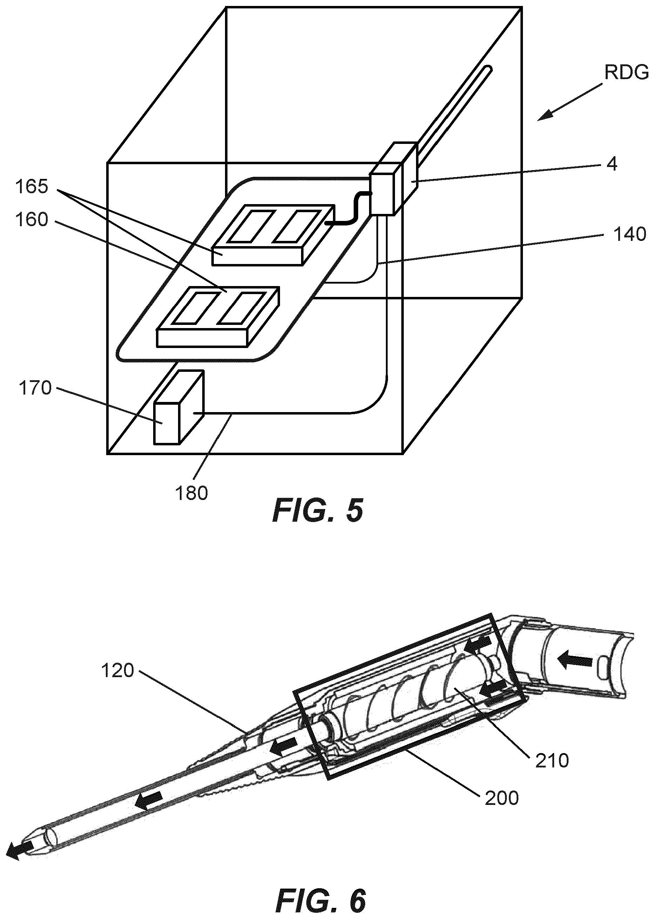

Fig. 5 zeigt eine schematische Darstellung eines in einem Reinigungs-/DesinfektionsGerät dauerhaft verbauten Moduls mit Ladepads bei einer Energiegewinnungsvorrichtung gemäß einem Ausführungsbeispiel (Stand Alone Variante).

Gemäß dem in Fig. 5 gezeigten Ausführungsbeispiel ist ein Modul 100 (eine Energiegewinnungsvorrichtung 4) dauerhaft in einem RDG verbaut und mit zumindest einem Ladepad 160, welches für Spritzwasser durchlässig ausgeführt sein kann, verbunden. Über dem zumindest einen Ladepad 160 können dann in Siebkörben 165 gelagerte Produkte, Systeme und/oder Instrumentarien mit integrierten oder angekoppelten, wechselbaren Energiespeichern mit und ohne Durchspülung induktiv geladen werden. Ebenso können beispielsweise nicht durchspülbare Einzelprodukte 170 mit Energiespeicher mittels eines (Lade)Kabels 180 direkt an die Energiegewinnungsvorrichtung 4 angeschlossen sein und geladen werden.

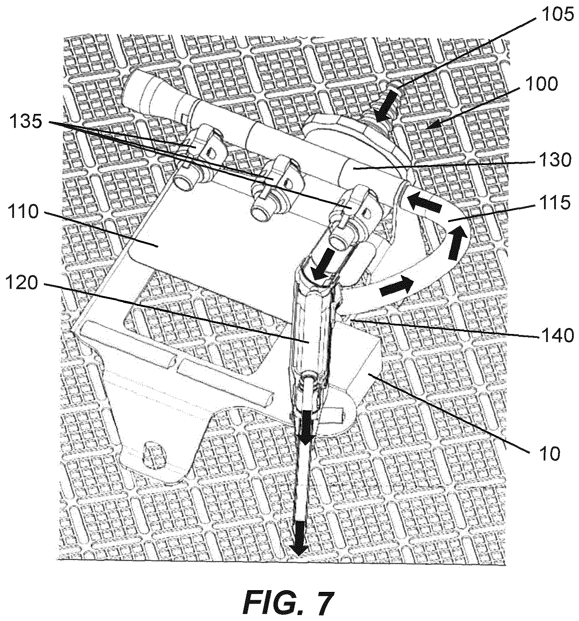

Fig. 6 zeigt als weitere Modifikation eine schematische Darstellung einer Nutzung eines in einem Produkt 120 verbauten, durchspülten Elektromotors 200 als Generator zum Laden eines Energiespeichers (nicht dargestellt) des Produkts mit im Generatorbetrieb erzeugter Energie bei einer Energiegewinnungsvorrichtung bzw. einem Energiegewinnungsmodul gemäß einem anderen Ausführungsbeispiel. Fig. 7 zeigt eine schematische Darstellung der Nutzung des in einem Produkt verbauten Elektromotors als Generator zum Laden des Energiespeichers des Produkts mit im Generatorbetrieb erzeugter Energie bei einer Energiegewinnungsvorrichtung gemäß dem anderen Ausführungsbeispiel. Gemäß Fig. 6 und Fig. 7 ist sozusagen die Energiegewinnungsvorrichtung 4 in das Produkt, System oder Instrument verlagert bzw. werden die dort entsprechend bereits vorhandenen technischen Elemente zur Energiegewinnung in situ genutzt.

Der Elektromotor 200 wird während des Durchspülens im RDG als Generator eingesetzt. Ein mit einer geeigneten Geometrie (beispielsweise schneckenwellenförmig) ausgeprägter Rotor 210 des Elektromotors 200 erzeugt in Zusammenwirkung mit einem Stator des Elektromotors elektrischen Strom. Der erzeugte elektrische Strom wird dazu genutzt, über eine Induktionsvorrichtung 110, alternativ vermittels eines Ladekabels (nicht dargestellt), den Energiespeicher zu laden. Erforderlichenfalls wird eine elektronische Steuerung 10 eingesetzt. Im Übrigen entspricht das in Fig. 7 gezeigte Modul 100 mit Ausnahme der Energiegewinnungsvorrichtung 4, dessen Funktion in diesem Ausführungsbeispiel von dem durchspülten und während der Durchspülung im Generatorbetrieb betriebenen Elektromotor 200 übernommen wird, dem in Fig. 2 gezeigten Modul 100.

Wie vorstehend beschrieben wurde, ist eine Energiegewinnungsvorrichtung für einen aufladbaren Energiespeicher an einer von einem Fluid durchströmten Fluidleitung eines bestehenden Fluidkreislaufs in einem Gerät angeordnet. Der Fluidstrom in der Fluidleitung dient primär einem vorbestimmten Zweck, der sich von dem der Energiegewinnung unterscheidet. Die Energiegewinnungsvorrichtung ist dazu angeordnet, aus der Bewegungsenergie des Fluidstroms Energie zur Aufladung des aufladbaren Energiespeichers zu gewinnen. Ein in das Gerät einbringbares Modul kann die Energiegewinnungsvorrichtung beinhalten. Das Gerät kann praxisnah ein Reinigungs-/Desinfektionsgerät (RDG) sein zur Aufbereitung medizinischer Instrumente, Systeme und/oder Produkte, wobei die medizinischen Instrumente, Systeme und/oder Produkte zumindest ein Handstück mit einem an dieses ankoppelbaren und/oder in dieses integrierten, aufladbaren Energiespeicher umfassen, wobei der Energiespeicher als Akkumulator oder als Powercell konfiguriert ist und dazu ausgelegt ist, zusammen mit dem Handstück in dem Reinigungs-/Desinfektionsgerät aufbereitet zu werden.

Es versteht sich, dass die Erfindung nicht auf das vorstehend beschriebene Ausführungsbeispiel beschränkt ist, sondern Änderungen, Modifikationen, Kombinationen und äquivalente Anordnungen im Rahmen des anspruchsgemäß definierten Schutzumfangs ebenfalls von der Erfindung umfasst sind, wobei sich dem Fachmann solche Änderungen, Modifikationen, Kombinationen und äquivalente Anordnungen ohne Weiteres ergeben.

## Patentansprüche

1. Modul (100) zur Verwendung in einem Aufbereitungsprozess für medizinische Produkte, Systeme und/oder Instrumente (120) mit einem integrierten oder ankoppelbaren, wechselbaren und aufladbaren Energiespeicher, wobei das Modul (100) dazu konfiguriert ist, während des Aufbereitungsprozesses in einem Reinigungs-/Desinfektionsgerät (RDG) als einer Aufbereitungsvorrichtung gehalten oder gelagert zu werden, und wobei das Modul (100) hat:
einen Anschluss (105) zur Verbindung des Moduls (100) mit einem bestehenden Fluidkreislauf des Reinigungs-/Desinfektionsgeräts (RDG);
eine Leitungsverbindung als eine Fluidleitung (115) zur Führung eines Fluidstroms als an dem Anschluss (105) in das Modul (100) einströmenden Fluids durch das Modul (100);
einen Portabschnitt (130);
zumindest einen Anschlussport (135) für das zumindest eine medizinische Produkt, System und/oder Instrument (120), der an dem Portabschnitt (130) angeordnet ist und dazu konfiguriert ist, das zumindest eine medizinische Produkt, System und/oder Instrument (120) derart zu halten, dass dieses von über den Anschlussport (135) einströmendem Fluid durchspült werden kann;
eine Energiegewinnungsvorrichtung (4), die dazu konfiguriert ist, an oder in der von dem Fluidstrom durchströmten Fluidleitung (115) des Fluidkreislaufs des Reinigungs-/Desinfektionsgeräts (RDG) angeordnet zu sein, und dazu angeordnet ist, aus der Bewegungsenergie des Fluidstroms elektrische Energie zur Aufladung des aufladbaren Energiespeichers zu gewinnen, wobei der Fluidstrom in der Fluidleitung (115) primär einem vorbestimmten Zweck dient, der sich von dem der Energiegewinnung unterscheidet; und
eine Induktionsvorrichtung (110), die in einem Bereich angeordnet ist, in dem der Energiespeicher des an dem Anschlussport (135) angeschlossenen zumindest einen medizinischen Produkts, Systems und/oder Instruments (120) im angeschlossenen Zustand zu liegen kommt, und dazu konfiguriert ist, den Energiespeicher vermittels von der Energiegewinnungsvorrichtung (4) aus dem Fluidstrom geernteter Energie während des Betriebs des Reinigungs-/Desinfektionsgeräts (RDG) zu laden.

2. Modul (100) nach Anspruch 1, **gekennzeichnet durch**:
eine Umwandlungseinheit (6) zur Umwandlung des Fluiddrucks des Fluidstroms in eine Rotationsbewegung; und
eine Generatoreinheit (8) zur Umwandlung der Rotationsbewegung in die erzeugte Energie.

3. Modul (100) nach Anspruch 2, **dadurch gekennzeichnet, dass** die Umwandlungseinheit (6) zur Umwandlung des Fluiddrucks in eine Rotationsbewegung ein Rotorelement mit einer vorbestimmten Geometrie beinhaltet, das in einem Gehäuse der Energiegewinnungsvorrichtung (4) und in dem Fluidstrom liegend drehbar gelagert ist.

4. Modul (100) nach Anspruch 3, **dadurch gekennzeichnet, dass** die vorbestimmte Geometrie schaufelradförmig oder schneckenförmig erzeugt ist.

5. Modul (100) nach einem der Ansprüche 2 bis 4, **dadurch gekennzeichnet, dass** die Umwandlungseinheit (6) zur Umwandlung des Fluiddrucks in eine Rotationsbewegung ein während eines Durchspülvorgangs von der Fluidströmung angetriebener, als die Generatoreinheit (8) arbeitender Elektromotor ist, der einen eine vorbestimmte Geometrie aufweisenden Rotor beinhaltet und dazu konfiguriert ist, in Zusammenwirkung mit einem Statorelement elektrischen Strom zu erzeugen, mittels dem der Energiespeicher vermittels der Induktionseinrichtung (110) zum induktiven Laden des Energiespeichers aufladbar ist.

6. Modul (100) nach einem der vorangehenden Ansprüche, **dadurch gekennzeichnet, dass** die Energiegewinnungsvorrichtung (4) eine elektronische Steuerung (10) beinhaltet, die in einem Gehäuse der Energiegewinnungsvorrichtung (4) auf einer Leiterplatte angeordnet ist.

7. Modul (100) nach Anspruch 6, **dadurch gekennzeichnet, dass**:
- die Energiegewinnungsvorrichtung (4) eine Umwandlungseinheit (6) zur Umwandlung des Fluiddrucks des Fluidstroms in eine Rotationsbewegung;
eine Generatoreinheit (8) zur Umwandlung der Rotationsbewegung in die erzeugte Energie hat; und
- die elektronische Steuerung (10) dazu konfiguriert ist, einen Füllstand des Energiespeichers zu erfassen und bei Erfassen eines vorbestimmten Füllstands einen Bypass zu öffnen, wobei der Bypass dazu angeordnet ist, im geöffneten Zustand den Fluidstrom an der Umwandlungseinheit (6) vorbeizuführen, und wobei in diesem Fall die Umwandlungseinheit (6) dazu konfiguriert ist, sowohl ihre Rotation als auch die Erzeugung der Energie zur Aufladung des Energiespeichers zu beenden.

8. Modul (100) nach Anspruch 2, **dadurch gekennzeichnet, dass** die von der Generatoreinheit erzeugte Energie vermittels der eine Induktionsleitung umfassenden flexiblen Leiterplatte als der Induktionsvorrichtung (110) an den Energiespeicher übertragbar ist.

9. Modul (100) nach Anspruch 1, **dadurch gekennzeichnet, dass** das Modul (100) eine Vorrichtung zur Ermittlung eines Ladezustands des angebundenen Energiespeichers und eine Datenübertragungsschnittstelle, über welche das Modul (100) auslesbar ist oder den Ladezustand nach außen hin kommuniziert, aufweist.

10. Modul (100) nach einem der vorangehenden Ansprüche, **dadurch gekennzeichnet, dass** die medizinischen Instrumente, Systeme und/oder Produkte (120) zumindest ein Handstück (120) mit einem an dieses ankoppelbaren und/oder in dieses integrierten, aufladbaren Energiespeicher umfassen, wobei der Energiespeicher als Akkumulator oder als Powercell konfiguriert ist und dazu ausgelegt ist, zusammen mit dem Handstück (120) in dem Reinigungs-/Desinfektionsgerät (RDG) aufbereitet zu werden.

11. Reinigungs-/Desinfektionsgerät (RDG) zur Aufbereitung medizinischer Instrumente, Systeme und/oder Produkte (120) mit einem Modul (100) nach einem der vorangehenden Ansprüche mit einer Energiegewinnungsvorrichtung (4), wobei die medizinischen Instrumente, Systeme und/oder Produkte (120) zumindest ein Handstück (120) mit einem an dieses ankoppelbaren und/oder in dieses integrierten, aufladbaren Energiespeicher umfassen, wobei der Energiespeicher als Akkumulator oder Powercell konfiguriert ist und dazu ausgelegt ist, zusammen mit dem Handstück (120) in dem Reinigungs-/Desinfektionsgerät aufbereitet (RDG) zu werden,
**dadurch gekennzeichnet, dass**:
a) die Energiegewinnungsvorrichtung (4) des Moduls (100) dazu konfiguriert ist, an einer vorbestimmten Lagerung oder einem vorbestimmten Siebkorb des Reinigungs-/Desinfektionsgeräts (RDG) gehalten zu werden, oder
b) das Modul (100) als ein an einem Spülrack des Reinigungs-/Desinfektionsgeräts (RDG) integriertes Modul (100) ausgebildet ist, oder
c) das Modul (100) als ein an einer vorbestimmten Lagerung des Reinigungs-/Desinfektionsgeräts (RDG) integriertes Modul (100) mit der Induktionsvorrichtung (110) zur drahtlosen Übertragung von Energie mittels Induktion zum Laden eines in dem Reinigungs-/Desinfektionsgerät (RDG) aufgenommenen und mit zumindest einem Energiespeicher ausgestatteten Instrumentariums (120) oder eines an einem Schlauchabschnitt (2) des Reinigungs-/Desinfektionsgerät (RDG) angeschlossenen Produkts ausgebildet ist.

12. Reinigungs-/Desinfektionsgerät (RDG) nach dem direkt vorstehenden auf das Reinigungs-/Desinfektionsgerät (RDG) gerichteten Anspruch, **dadurch gekennzeichnet, dass** das Modul (100) dauerhaft in dem Reinigungs-/Desinfektionsgerät (RDG) verbaut und mit zumindest einem spritzwasserdurchlässigen Ladepad verbunden ist, wobei das Ladepad dazu konfiguriert ist, zumindest ein Produkt mit zumindest einem Energiespeicher, das über dem Ladepad gelagert ist, induktiv mit der von dem Modul (100) erzeugten Energie zu laden.

13. Verfahren zum Aufladen eines Energiespeichers eines chirurgischen Instruments (120) mit folgenden Verfahrensschritten:
- Anschließen oder Einlegen des chirurgischen Instruments (120) mit darin verbautem Energiespeicher an oder in ein Reinigungs- und/oder Desinfektionsgerät (RDG),
- Anschließen des Anschlusses (105) eines Moduls (100) nach einem der vorangehenden auf das Modul (100) gerichteten Ansprüche mit einer Fluidströmungsenergie in elektrische Energie umwandelnden Energiegewinnungsvorrichtung (4) an das Reinigungs- und/oder Desinfektionsgerät (RDG), vorzugsweise an dessen Fluideingangs- oder Fluidausgangsanschluss,
- Anschließen des Energiespeichers an die Energiegewinnungsvorrichtung (4), wobei der Anschließschritt bezüglich des Energiespeichers auch vor dem Anschließschritt bezüglich der Energiegewinnungsvorrichtung (4) erfolgen kann,
- bestimmungsgemäßes Betreiben des Reinigungs- und/oder Desinfektionsgeräts (RDG).

## Claims

1. A module (100) for use in a processing process for medical products, systems and/or instruments (120) having an integrated or connectable, exchangeable and chargeable energy storage device, the module (100) being configured to be held or supported during the processing process in a washer disinfector (RDG) as a processing device, the module (100) comprising:
a connection (105) for connecting the module (100) to an existing fluid circuit of the washer disinfector (RDG),
a conduit as a fluid conduit (115) for guiding a fluid flow as fluid flowing through the module (100) into the module (100) at the connection (100);
a port portion (130);
at least one connection port (135) for the at least one medical product, system and/or instrument (120) provided at the port portion (130) and configured to hold the medical product, system and/or instrument (120) such that the medical product, system and/or instrument (120) can be flushed by fluid flowing in through the connection port (135);
an energy production device (4) configured to be arranged on or in the fluid conduit (115) of the fluid circuit of the washer disinfector (RDG) through which the fluid flow flows, and arranged to produce electric energy from kinetic energy of the fluid flow for charging the rechargeable energy storage device, wherein the fluid flow in the fluid conduit (115) primarily serves a predetermined purpose different from that of energy generation; and
an inductor (110) arranged in an area in which the energy storage device of the at least one medical product, system and/or instrument (120) connected to the connection port (135) is located in a connected state, and which is configured to charge the energy storage device via energy harvested from the fluid flow via the energy production device (4), during operation of the washer disinfector (RDG).

2. The module (100) according to claim 1, **characterized by**:
a conversion unit (6) for converting the fluid pressure of the fluid flow into a rotational movement; and
a generator unit (8) for converting the rotational movement into produced energy.

3. The module (100) according to claim 2, **characterized in that** the conversion unit (6) for converting the fluid pressure into the rotational movement comprises a rotor element with a predetermined geometry, which is rotatably mounted in a housing of the energy production device (4) and which is lying in the fluid flow.

4. The module (100) according to claim 3, **characterized in that** the predetermined geometry is formed in a vane shape or in a helical shape.

5. The module (100) according to one of the claims 2 to 4, **characterized in that**, for converting the fluid pressure into a rotational movement, the conversion unit (6) is an electric motor driven by the fluid flow during a flushing process and opering as the generator unit (8), the electric motor including the rotor having a predetermined geometry and being configured to produce electric current in cooperation with a stator element by which the energy storage device is chargeable via the inductor (110) for inductive charging of the energy storage device.

6. The module (100) according to one of the preceding claims, **characterized in that** the energy production device (4) includes an electronic control (10) arranged on a circuit board in a housing of the energy production device (4).

7. The module (100) according to claim 6, **characterized in that**:
- the energy production device (4) has a conversion unit (6) for converting the fluid pressure of the fluid flow into a rotational movement; a generator unit (8) for converting the rotational movement into produced energy; and
- the electronic control (10) is configured to detect a filling level of the energy storage device and to open a bypass upon detection of a predetermined filling level, wherein the bypass is arranged to guide the fluid flow in an opened state past the conversion unit (6), and wherein in this case, the conversion unit (6) is configured to stop rotation and stop production of energy for charging the energy storage device.

8. The module (100) according to claim 2, **characterized in that** the energy produced by the generator unit is transferable to the energy storage device via the flexible circuit board as the inductor (110) comprising an induction line.

9. The module (100) according to claim 1, **characterized in that** the module (100) comprises a device for determining a charging state of the linked energy storage device and a data transmission interface by which the module (100) can be read or externally communicates the charging state.

10. The module (100) according to one of the preceding claims, **characterized in that** the medical instruments, systems and/or products (120) comprise at least one handpiece (120) with the energy storage device which is connectable thereto and/or is integrated therein, wherein the energy storage device is configured as a rechargeable battery or as a power cell and is configured to be processed together with the handpiece (120) in the washer disinfector (RDG).

11. The washer disinfector (RDG) for processing medical instruments, systems, and/or products (120) with a module (100) according to one of the preceding claims with an energy production device (4), wherein the medical instruments, systems, and/or products (120) comprise at least one handpiece (120) with a rechargeable energy storage device which is connectable thereto and/or is integrated therein, wherein the energy storage device configured as a rechargeable battery or power cell and configured to be processed in the washer-disinfector (RDG) together with the handpiece (120),
**characterized in that**:
a) the energy generation device (4) of the module (100) configured to be held at a predetermined support or a predetermined sieve basket of the washer disinfector (RDG), or
b) the module (100) is configured as a module (100) integrated into a rinsing rack of the washer disinfector (RDG), or
c) the module (100) is configured as a module (100) integrated into a predetermined storage location of the washer disinfector (RDG) with the induction device (110) for wireless transmission of energy via induction for charging an instrument set (120) accommodated in the washer disinfector (RDG) and equipped with at least one energy storage device, or a product connected to a hose section (2) of the washer disinfector (RDG).

12. The washer disinfector (RDG) according to the immediately preceding claim directed to the washer disinfector (RDG), **characterized in that** the module (100) is permanently installed in the washer disinfector (RDG) and connected to at least one splash-proof charging pad, wherein the charging pad is configured to inductively charge at least one product with at least one energy storage device stored above the charging pad with the energy generated by the module (100).

13. A method of charging an energy storage device of a surgical instrument (120) comprising the following steps:
- connecting or inserting the surgical instrument (120) with the energy storage device installed therein to or into a washing and/or disinfection device;
- connecting the connection (105) of a module (100) according to one of the preceding claims directed to the module (100) to the washer disinfector (RDG) with an energy production device (4) that converts fluid flow energy into electrical energy, preferably to the washer disinfector's (RDG) fluid inlet or fluid outlet connection,
- connecting the energy storage device to the energy production device (4), wherein the connection step with regard to the energy storage device can also be performed before the connection step with regard to the energy generation device (4),
- operating the washer disinfector (RDG) as intended.

## Revendications

1. Module (100) destiné à être utilisé dans un processus de préparation pour des produits, systèmes et/ou instruments (120) médicaux avec un dispositif de stockage d'énergie intégré ou pouvant être couplé, interchangé et rechargé, dans lequel le module (100) est configuré afin d'être, pendant le processus de préparation, maintenu ou logé dans un appareil de nettoyage/de désinfection (RDG) comme dispositif de préparation, et dans lequel le module (100) présente :
un raccord (105) pour lier le module (100) à un circuit de fluide existant de l'appareil de nettoyage/de désinfection (RDG) ;
une liaison de conduite comme conduite de fluide (115) pour guider un écoulement de fluide comme fluide entrant au niveau du raccord (105) dans le module (100) par le module (100) ;
une section de port (130) ;
au moins un port de raccordement (135) pour le au moins un produit, système et/ou instrument (120) médical qui est agencé au niveau de la section de port (130) et est configuré afin de maintenir le au moins un produit, système et/ou instrument (120) médical de telle manière que celui-ci puisse être rincé par du fluide entrant par le biais du port de raccordement (135) ;
un dispositif de production d'énergie (4) qui est configuré afin d'être agencé au niveau de ou dans la conduite de fluide (115) traversée par le écoulement de fluide du circuit de fluide de l'appareil de nettoyage/de désinfection (RDG) et est agencé afin de produire de l'énergie électrique pour charger le dispositif de stockage d'énergie rechargeable à partir de l'énergie cinétique du écoulement de fluide, dans lequel le écoulement de fluide dans la conduite de fluide (115) sert principalement à un but prédéterminé qui se distingue de celui de la production d'énergie ; et
un dispositif d'induction (110) qui est agencé dans une zone dans laquelle le dispositif de stockage d'énergie du produit, système et/ou instrument (120) médical raccordé au port de raccordement (135) vient se poser dans l'état raccordé, et est configuré afin de charger le dispositif de stockage d'énergie par l'intermédiaire de l'énergie récoltée par le dispositif de production d'énergie (4) à partir du écoulement de fluide pendant le fonctionnement de l'appareil de nettoyage/de désinfection (RDG).

2. Module (100) selon la revendication 1, **caractérisé par** :
une unité de conversion (6) pour convertir la pression de fluide du écoulement de fluide en un mouvement de rotation ; et
une unité de générateur (8) pour convertir le mouvement de rotation en énergie générée.

3. Module (100) selon la revendication 2, **caractérisé en ce que** l'unité de conversion (6) pour convertir la pression de fluide en un mouvement de rotation contient un élément de rotor avec une géométrie prédéterminée qui est logé de manière rotative dans un boîtier du dispositif de production d'énergie (4) et se trouvant dans l'écoulement de fluide.

4. Module (100) selon la revendication 3, **caractérisé en ce que** la géométrie prédéterminée est générée en forme de roue à aubes ou en forme d'hélice.

5. Module (100) selon l'une quelconque des revendications 2 à 4, **caractérisé en ce que** l'unité de conversion (6) pour convertir la pression de fluide en un mouvement de rotation est un moteur électrique travaillant comme l'unité de générateur (8), entraîné par l'écoulement de fluide pendant un processus de rinçage, moteur qui contient un rotor présentant une géométrie prédéterminée et est configuré afin de générer un courant électrique en coopération avec un élément de stator, courant au moyen duquel le dispositif de stockage d'énergie est rechargeable à l'aide du dispositif d'induction (110) pour la charge inductive du dispositif de stockage d'énergie.

6. Module (100) selon l'une quelconque des revendications précédentes, **caractérisé en ce que** le dispositif de production d'énergie (4) contient un dispositif de commande (10) électronique qui est agencé dans un boîtier du dispositif de production d'énergie (4) sur une carte de circuit imprimé.

7. Module (100) selon la revendication 6, **caractérisé en ce que** :
- le dispositif de production d'énergie (4) présente une unité de conversion (6) pour convertir la pression de fluide de l'écoulement de fluide en un mouvement de rotation ;
une unité de générateur (8) pour convertir le mouvement de rotation en énergie générée ; et
- le dispositif de commande (10) électronique est configuré afin de détecter un niveau de remplissage de l'accumulateur d'énergie et, si un niveau de remplissage prédéterminé est détecté, d'ouvrir une dérivation, dans lequel la dérivation est agencée afin de faire passer à l'état ouvert l'écoulement de fluide devant l'unité de conversion (6), et dans lequel dans ce cas l'unité de conversion (6) est configurée afin de terminer non seulement sa rotation mais aussi la génération de l'énergie pour charger le dispositif de stockage d'énergie.

8. Module (100) selon la revendication 2, **caractérisé en ce que** l'énergie générée par l'unité de générateur peut être transmise au dispositif de stockage d'énergie par l'intermédiaire de la carte de circuit imprimé flexible comprenant une conduite d'induction en tant que dispositif d'induction (110).

9. Module (100) selon la revendication 1, **caractérisé en ce que** le module (100) présente un dispositif de détermination d'un état de charge du dispositif de stockage d'énergie lié et une interface de transmission de données par le biais de laquelle le module (100) peut être lu ou communique l'état de charge vers l'extérieur.

10. Module (100) selon l'une quelconque des revendications précédentes, **caractérisé en ce que** les instruments, systèmes et/ou produits (120) médicaux comprennent au moins une pièce à main (120) avec un dispositif de stockage d'énergie rechargeable pouvant être couplé à celle-ci et/ou intégré dans celle-ci, dans lequel le dispositif de stockage d'énergie est configuré comme accumulateur ou comme cellule de puissance et est conçu afin d'être préparé conjointement avec la pièce à main (120) dans l'appareil de nettoyage/de désinfection (RDG).

11. Appareil de nettoyage/de désinfection (RDG) destiné à préparer des instruments, systèmes et/ou produits (120) médicaux avec un module (100) selon l'une quelconque des revendications précédentes avec un dispositif de production d'énergie (4), dans lequel les instruments, systèmes et/ou produits (120) médicaux comprennent au moins une pièce à main (120) avec un dispositif de stockage d'énergie rechargeable pouvant être couplé à celle-ci et/ou intégré dans celle-ci, dans lequel le dispositif de stockage d'énergie est configuré comme accumulateur ou cellule de puissance et est conçu afin d'être préparé conjointement avec la pièce à main (120) dans l'appareil de nettoyage/de désinfection (RDG).
**caractérisé en ce que** :
a) le dispositif de production d'énergie (4) du module (100) est configuré afin d'être maintenu au niveau d'un logement prédéterminé ou d'une crépine prédéterminée de l'appareil de nettoyage/de désinfection (RDG) ou
b) le module (100) est formé comme un module (100) intégré au niveau d'un rack de rinçage de l'appareil de nettoyage/de désinfection (RGD) ou
c) le module (100) est formé comme un module (100) intégré au niveau d'un logement prédéterminé de l'appareil de nettoyage/de désinfection (RDG) avec le dispositif d'induction (110) pour la transmission sans fil d'énergie par induction pour charger un instrument (120) logé dans l'appareil de nettoyage/de désinfection (RDG) et équipé d'au moins un dispositif de stockage d'énergie ou d'un produit raccordé à une section de tuyau (2) de l'appareil de nettoyage/de désinfection (RDG).

12. Appareil de nettoyage/de désinfection (RDG) selon la revendication directement précédente traitant de l'appareil de nettoyage/de désinfection (RDG), **caractérisé en ce que** le module (100) est durablement monté dans l'appareil de nettoyage/de désinfection (RGD) et est relié à au moins un pad de charge perméable à l'eau pulvérisée, dans lequel le pad de charge est configuré afin de charger au moins un produit avec au moins un dispositif de stockage d'énergie, qui est logé au-dessus du pad de charge, de manière inductive avec l'énergie générée par le module (100).

13. Procédé pour charger un dispositif de stockage d'énergie d'un instrument (120) chirurgical avec les étapes de procédé suivantes :
- le raccordement ou la pose de l'instrument (120) chirurgical avec un dispositif de stockage d'énergie intégré dans celui-ci au niveau de ou dans un appareil de nettoyage/de désinfection (RDG),
- le raccordement du raccord (105) d'un module (100) selon l'une quelconque des revendications précédentes traitant du module (100) avec un dispositif de production d'énergie (4) convertissant de l'énergie de écoulement de fluide en énergie électrique à l'appareil de nettoyage et/ou de désinfection (RDG), de préférence au niveau de son raccord d'entrée de fluide ou de sortie de fluide,
- le raccordement du dispositif de stockage d'énergie au dispositif de production d'énergie (4), dans lequel l'étape de raccordement relative au dispositif de stockage d'énergie peut aussi être effectuée avant l'étape de raccordement relative au dispositif de production d'énergie (4),
- le fonctionnement conforme de l'appareil de nettoyage et/ou de désinfection (RDG).
